# EUROPEAN PATENT APPLICATION

(11) **EP 1 281 352 A1**
(43) Date of publication of application: **05.02.2003**
(21) Application number: 02255253.3
(22) Date of filing: 26.07.2002
(51) Int. Cl.: A61B 5/00, A61B 5/15

(54) **Test strip for analyte concentration determination of a physiological sample**

(30) Priority: 01.08.2001 US 919981
(71) Applicant: Lifescan, Inc., Milpitas, CA 95035 (US)
(72) Inventor: Yuzhakov, Vadim Vladimirovich, San Jose, California 95127 (US); Mcallister, Devin V., San Jose, California 95112 (US)
(74) Representative: Mercer, Christopher Paul

(57) **Abstract**

Test strips (2) are provided for use in the determination of the concentration of at least one target analyte in a physiological sample. The test strips include a plurality of skin-piercing elements (18) affixed thereto. The plurality of skin-piercing elements affixed to the test strip is inserted into the skin, the physiological sample is transferred to the test strip and the concentration of an analyte is determined. The test strips are configured to be received into a meter. The test strips find use in a variety of different applications, particularly in the determination of glucose concentrations.

## Description

### INTRODUCTION

### FIELD OF THE INVENTION

The field of this invention is analyte concentration determination, particularly physiological sample concentration determination and more particularly glucose concentration determination.

### BACKGROUND OF THE INVENTION

Analyte concentration determination in physiological samples is of ever increasing importance to today's society. Such assays find use in a variety of application settings, including clinical laboratory testing, home testing, etc., where the results of such testing play a prominent role in the diagnosis and management of a variety of disease conditions. Analytes of interest include glucose for diabetes management, cholesterol for monitoring cardiovascular conditions, and the like. In response to this growing importance of analyte concentration determination, a variety of analyte concentration determination protocols and devices for both clinical and home testing have been developed.

However, to determine the concentration of an analyte in a physiological sample, a physiological sample must first be obtained. Obtaining the sample often involves cumbersome and complicated devices which may not be easy to use or may be costly to manufacture. Furthermore, the procedure for obtaining the sample may also be painful. For example, pain is often associated with the size of the needle used to obtain the physiological sample and the depth to which the needle is inserted. Depending on the analyte and test employed, a relatively large, single needle or the like is often used to extract the requisite amount of sample. Furthermore, the process may involve a multitude of steps. For example, once obtained, a patient may be required to activate a skin-piercing mechanism and then activate a sample collection mechanism and the sample must then be transferred to a testing device, *e*.*g*., a test strip or the like, and then oftentimes the test strip is then transferred to a measuring device such as a meter. Because of these disadvantages, it is not uncommon for patients who require frequent monitoring of an analyte to simply avoid monitoring the analyte of interest. With diabetics, for example, the failure to measure their glucose level on a prescribed basis results in a lack of information necessary to properly control the level of glucose. Uncontrolled glucose levels can be very dangerous and even life threatening.

The simplest devices for obtaining a sample are not integrated with a testing device at all, but are simply single needles or lances which are used to pierce the skin. However, these simple devices do not solve the problem of reducing the steps involved in the sampling and measurement procedures or minimizing pain. Furthermore, they may be costly to manufacture.

Attempts have been made to combine a lancing-type device with various other components involved in analyte concentration determination. For example, U.S. Patent No. 6,099,484 discloses a sampling device which includes a single needle associated with a spring mechanism, a capillary tube associated with a pusher and a test strip. An analyzer may also be mounted in the device for analyzing the sample. Accordingly, the single needle is displaced toward the skin surface by un-cocking a spring and then retracting it by another spring. A pusher is then displaced to push the capillary tube in communication with a sample and the pusher is then released and the fluid is transferred to a test strip.

U.S. Patent No. 5,820,570 discloses an apparatus which includes a base having a hollow needle and a cover having a membrane, whereby the base and cover are connected together at a hinge point. When in a closed position, the needle is in communication with the membrane and fluid can be drawn up through the needle and placed on the membrane of the cover.

While effective, there are drawbacks associated with each of the above devices and techniques. For example, the devices disclosed in the aforementioned patents are complex, thus decreasing ease-of-use and increasing manufacturing costs. Furthermore, as described, a single needle design may be associated with increased pain because the single needle must be large enough to extract the requisite sample size. Still further, in regards to the '484 patent, the steps of activating and retracting a needle and then activating and retracting a capillary tube adds still more user interaction and decreases ease-of-use.

As such, there is continued interest in the development of new devices and methods for use in the determination of analyte concentrations in a physiological sample. Of particular interest would be the development of such devices, and methods of use thereof, that are efficient, involve minimal pain, are simple to use and which may be used with various analyte concentration determination systems.

### SUMMARY OF THE INVENTION

Test strips and methods are provided for use in the determination of the concentration of at least one target analyte in a physiological sample. The subject test strips include a plurality of skin-piercing elements affixed thereto. In the subject methods, the plurality of skin-piercing elements affixed to the test strip is inserted into the skin, physiological sample is transferred to the test strip and the concentration of an analyte is determined. The subject test strips and methods find use in a variety of different applications, particularly in the determination of glucose concentrations.

### BRIEF DESCRIPTIONS OF THE DRAWINGS

Figure 1A provides an exploded view of an exemplary test strip according to the present invention having a plurality of hollow skin-piercing elements affixed to the test strip in a substantially transverse position. Figure 1B provides an enlarged view of a skin-piercing element according Figure 1A having an inner lumen.

Figure 2A provides an exploded view of a test strip according to the present invention having a plurality of solid skin-piercing elements affixed to the test strip in a substantially transverse position and which plurality is associated with at least one fluid pathway. Figure 2B provides an isometric view of a colorimetric test strip having solid skin-piercing elements. Figure 2C provides an enlarged view of the micro-needles of Figs. 2A and 2B.

Figure 3 provides another embodiment of a test strip according to the present invention having a plurality of skin-piercing elements affixed to the test strip in a substantially parallel position.

Figures 4A and 4B provide yet another embodiment of a test strip of the present invention having a plurality of skin-piercing elements configured as two spaced apart, parallel sets of skin-piercing elements, where each set is formed by a respective electrode.

Figures 5A illustrates an embodiment of an electrochemical meter of the present invention for use with the test strips of the present invention. Figure 5B illustrates an embodiment of an optical meter of the present invention for use with the test strips of the present invention.

### DETAILED DESCRIPTION OF THE INVENTION

Test strips and methods are provided for use in the determination of the concentration of at least one target analyte in a physiological sample. The subject test strips include a plurality of skin-piercing elements affixed thereto. In the subject methods, the plurality of skin-piercing elements affixed to the test strip is inserted into the skin, physiological sample is transferred to the test strip and the concentration of an analyte is determined. The subject test strips and methods find use in a variety of different applications, particularly in the determination of glucose concentrations. In further describing the subject invention, the subject devices will be described first, followed by a review of the subject methods for use in practicing the subject devices.

Before the present invention is described, it is to be understood that this invention is not limited to the particular embodiments described, as such may, of course, vary. It is also to be understood that the terminology used herein is for the purpose of describing particular embodiments only, and is not intended to be limiting, since the scope of the present invention will be limited only by the appended claims.

Where a range of values is provided, it is understood that each intervening value, to the tenth of the unit of the lower limit unless the context clearly dictates otherwise, between the upper and lower limit of that range and any other stated or intervening value in that stated range is encompassed within the invention. The upper and lower limits of these smaller ranges may independently be included in the smaller ranges is also encompassed within the invention, subject to any specifically excluded limit in the stated range. Where the stated range includes one or both of the limits, ranges excluding either both of those included limits are also included in the invention.

Unless defined otherwise, all technical and scientific terms used herein have the same meaning as commonly understood by one of ordinary skill in the art to which this invention belongs. Although any methods and materials similar or equivalent to those described herein can also be used in the practice or testing of the present invention, the preferred methods and materials are now described.

It must be noted that as used herein and in the appended claims, the singular forms "a", "an", and "the" include plural referents unless the context clearly dictates otherwise. Thus, for example, reference to "a test strip" includes a plurality of such test strips and reference to "the device" includes reference to one or more devices and equivalents thereof known to those skilled in the art, and so forth.

All publications mentioned herein are incorporated herein by reference to disclose and describe the methods and/or materials in connection with which the publications are cited. The publications discussed herein are provided solely for their disclosure prior to the filing date of the present application. Nothing herein is to be construed as an admission that the present invention is not entitled to antedate such publication by virtue of prior invention. Further, the dates of publication provided may be different from the actual publication dates which may need to be independently confirmed.

### DEVICES

As summarized above, the subject invention provides test strips for determining the concentration of an analyte in a physiological sample, where the test strips advantageously allow for the access and collection of the sample by way of a plurality of skin-piercing elements affixed directly to the test strip. The subject test strips find use in the determination of a wide variety of different analyte concentrations, where representative analytes include, but are not limited to, glucose, cholesterol, lactate, alcohol, and the like. In many embodiments, the subject test strips are used to determine the glucose concentration in a physiological sample, *e*.*g*., interstitial fluid, blood, blood fractions, constituents thereof, and the like.

While it is to be understood that a variety of different types of test strips may be suitable for use with the present invention, *e*.*g*., colorimetric or photometric and electrochemical, the subject invention will be described herein in reference to an electrochemical assay system, where such description is by way of example and not limitation. However, a description of a colorimetric test strip suitable for use with the present invention will be described first.

### Colorimetric Test Strips

The colorimetric or photometric (herein used interchangeably) reagent test strips employed in these embodiments of the subject invention are generally made up of at least the following components: a matrix for receiving a sample, a reagent composition that typically includes one or more members of an analyte oxidation signal producing system and a support element. The subject test strips are configured and adapted to be received in an automated meter, as described below, for automatically determining the concentration of an analyte.

The matrix that is employed in the subject test strips is an inert matrix which provides a support for the various members of the signal producing system, described infra, as well as the light absorbing or chromogenic product produced by the signal producing system, *i*.*e*., the indicator. The matrix is configured to provide a location for the physiological sample, *e*.*g*., blood, application and a location for the detection of the light-absorbing product produced by the indicator of the signal producing system. As such, the matrix is one that is permissive of aqueous fluid flow through it and provides sufficient void space for the chemical reactions of the signal producing system to take place. A number of different matrices have been developed for use in various analyte detection assays, which matrices may differ in terms of materials, dimensions and the like, where representative matrices include, but are not limited to, those described in U.S. Patent Nos.: 4,734,360; 4,900,666; 4,935,346; 5,059,394; 5,304,468; 5,306,623; 5,418,142; 5,426,032; 5,515,170; 5,526,120; 5,563,042; 5,620,863; 5,753,429; 5,573,452; 5,780,304; 5,789,255; 5,843,691; 5,846,486; 5,968,836 and 5,972,294; the disclosures of which are herein incorporated by reference. In principle, the nature of the porous matrix is not critical to the subject test strips and therefore is chosen with respect to the other factors, including the nature of the instrument which is used to read the test strip, convenience and the like. As such, the dimensions and porosity of the test strip may vary greatly, where the matrix may or may not have pores and/or a porosity gradient, *e*.*g*. with larger pores near or at the sample application region and smaller pores at the detection region. Materials from which the matrix may be fabricated vary, and include polymers, *e*.*g*. polysulfone, polyamides, cellulose or absorbent paper, and the like, where the material may or may not be functionalized to provide for covalent or non-covalent attachment of the various members of the signal producing system.

In addition to the matrix, the subject test strips further include one or more members of a signal producing system which produces a detectable product in response to the presence of analyte, which detectable product can be used to derive the amount of analyte present in the assayed sample. In the subject test strips, the one or more members of the signal producing system are associated, *e*.*g*., covalently or non-covalently attached to, at least a portion of (*i*.*e*., the detection region) the matrix, and in many embodiments to substantially all of the matrix.

The signal producing system is an analyte oxidation signal producing system. By analyte oxidation signal producing system is meant that in generating the detectable signal from which the analyte concentration in the sample is derived, the analyte is oxidized by a suitable enzyme to produce an oxidized form of the analyte and a corresponding or proportional amount of hydrogen peroxide. The hydrogen peroxide is then employed, in turn, to generate the detectable product from one or more indicator compounds, where the amount of detectable product generated by the signal measuring system, *i*.*e*. the signal, is then related to the amount of analyte in the initial sample. As such, the analyte oxidation signal producing systems present in the subject test strips are also correctly characterized as hydrogen peroxide based signal producing systems.

As indicated above, the hydrogen peroxide based signal producing systems include an enzyme that oxidizes the analyte and produces a corresponding amount of hydrogen peroxide, where by corresponding amount is meant that the amount of hydrogen peroxide that is produced is proportional to the amount of analyte present in the sample. The specific nature of this first enzyme necessarily depends on the nature of the analyte being assayed but is generally an oxidase. As such, the first enzyme may be: glucose oxidase (where the analyte is glucose); cholesterol oxidase (where the analyte is cholesterol); alcohol oxidase (where the analyte is alcohol); lactate oxidase (where the analyte is lactate) and the like. Other oxidizing enzymes for use with these and other analytes of interest are known to those of skill in the art and may also be employed. In those preferred embodiments where the reagent test strip is designed for the detection of glucose concentration, the first enzyme is glucose oxidase. The glucose oxidase may be obtained from any convenient source, *e*.*g*. a naturally occurring source such as Aspergillus niger or Penicillum, or recombinantly produced.

The second enzyme of the signal producing system is an enzyme that catalyzes the conversion of one or more indicator compounds into a detectable product in the presence of hydrogen peroxide, where the amount of detectable product that is produced by this reaction is proportional to the amount of hydrogen peroxide that is present. This second enzyme is generally a peroxidase, where suitable peroxidases include: horseradish peroxidase (HRP), soy peroxidase, recombinantly produced peroxidase and synthetic analogs having peroxidative activity and the like. See *e*.*g*., Y. Ci, F. Wang; Analytica Chimica Acta, 233 (1990), 299-302.

The indicator compound or compounds, *e.g.*, substrates, are ones that are either formed or decomposed by the hydrogen peroxide in the presence of the peroxidase to produce an indicator dye that absorbs light in a predetermined wavelength range. Preferably the indicator dye absorbs strongly at a wavelength different from that at which the sample or the testing reagent absorbs strongly. The oxidized form of the indicator may be a colored, faintly-colored, or colorless final product that evidences a change in color of the testing side of the membrane. That is to say, the testing reagent can indicate the presence of glucose in a sample by a colored area being bleached or, alternatively, by a colorless area developing color.

Indicator compounds that are useful in the present invention include both one- and two-component chromogenic substrates. One-component systems include aromatic amines, aromatic alcohols, azines, and benzidines, such as tetramethyl benzidine-HCl. Suitable two-component systems include those in which one component is MBTH, an MBTH derivative (see for example those disclosed in U.S. Patent Application Ser. No. 08/302,575, incorporated herein by reference), or 4-aminoantipyrine and the other component is an aromatic amine, aromatic alcohol, conjugated amine, conjugated alcohol or aromatic or aliphatic aldehyde. Exemplary two-component systems are 3-methyl-2-benzothiazolinone hydrazone hydrochloride (MBTH) combined with 3-dimethylaminobenzoic acid (DMAB); MBTH combined with 3,5-dichloro-2-hydroxybenzene-sulfonic acid (DCHBS); and 3-methyl-2-benzothiazolinonehydrazone N-sulfonyl benzenesulfonate monosodium (MBTHSB) combined with 8-anilino-1 naphthalene sulfonic acid ammonium (ANS). In certain embodiments, the dye couple MBTHSB-ANS is preferred.

In yet other embodiments, signal producing systems that produce a fluorescent detectable product (or detectable non- fluorescent substance, *e*.*g*. in a fluorescent background) may be employed, such as those described in: Kiyoshi Zaitsu, Yosuke Ohkura: New fluorogenic substrates for Horseradish Peroxidase: rapid and sensitive assay for hydrogen peroxide and the Peroxidase. Analytical Biochemistry (1980) 109, 109-113.

The matrix is attached to a support element. The support element may be of a material that is sufficiently rigid to be inserted into the meter without undue bending or kinking. In many embodiments, the support member is made of material such as polyolefins, *e*.*g*., polyethylene or polypropylene, polystyrene or polyesters.

As described above, the support element is configured to enable the test strip to be inserted into a meter. As such, the support element, and thus the test strip, is typically in the form of a substantially rectangular or square-like strip. Typically, the length of the support element is about 1 to 1000 mm, usually about 10 to 100 mm and more usually about 20 to 60 mm. Typically, the width of the support element is about 1 to 100 mm, usually about 1 to 10 mm and more usually about 5 to 7 mm. Typically, the height or thickness of the support element is about 0.01 to 1 mm, usually about 0.1 to 1 mm and more usually about 0.3 to 0.4 mm.

Generally, for colorimetric assays, the sample is allowed to react with the members of the signal producing system to produce a detectable product that is present in an amount proportional to the initial amount present in the sample. The amount of detectable product, *i*.*e*., signal produced by the signal producing system, is then determined and related to the amount of analyte in the initial sample. As described, in certain embodiments, automated meters that perform the above mentioned detection and relation steps are employed. The above described reaction, detection and relating steps, as well as instruments for performing the same, are further described in U.S. Patent Application Serial Nos. 4,734,360; 4,900,666; 4,935,346; 5,059,394; 5,304,468; 5,306,623; 5,418,142; 5,426,032; 5,515,170; 5,526,120; 5,563,042; 5,620,863; 5,753,429; 5,573,452; 5,780,304; 5,789,255; 5,843,691; 5,846,486; 5,968,836 and 5,972,294; the disclosures of which are herein incorporated by reference.

Examples of such colorimetric reagent test strips suitable for use with the subject invention include those described in U.S. Patent Nos.: 5,563,042; 5,753,452; 5,789,255, herein incorporated by reference.

### Electrochemical Test Strips

Generally, the electrochemical test strips of the subject invention are made up of two opposing metal electrodes separated by a thin spacer layer, where these components define a reaction area or zone and where skin-piercing elements are further associated with the reaction area. In many embodiments a redox reagent system is located in the reaction area or zone. The subject test strips are configured and adapted to be received in an automated meter, as described below, for automatically determining the concentration of an analyte.

In certain embodiments of these electrochemical test strips, the working and reference electrodes are generally configured in the form of elongated rectangular strips. Typically, the length of the electrodes ranges from about 1.9 to 4.5 cm, usually from about 2.0 to 2.8 cm. The width of the electrodes ranges from about 0.07 to 0.76 cm, usually from about 0.24 to 0.60 cm. The working and reference electrodes each have a thickness typically ranging from about 10 to 100 nm and usually from about 10 to 20 nm.

The working and reference electrodes are further characterized in that at least the surfaces of the electrodes that face the reaction area of the electrochemical cell in the strip is a metal, where metals of interest include palladium, gold, platinum, silver, iridium, carbon (conductive carbon ink), doped tin oxide, stainless steel and the like. In many embodiments, the metal is gold or palladium.

While in principle the entire electrode may be made of the metal, each of the electrodes is generally made up of an inert support material on the surface of which is present a thin layer of the metal component of the electrode. In these more common embodiments, the thickness of the inert backing material typically ranges from about 25 to 500, usually 50 to 400 µm, while the thickness of the metal layer typically ranges from about 10 to 100 nm and usually from about 10 to 40 nm, *e*.*g*., a sputtered metal layer. Any convenient inert backing material may be employed in the subject electrodes, where typically the material is a rigid material that is capable of providing structural support to the electrode and, in turn, the electrochemical test strip as a whole. Suitable materials that may be employed as the inert support material include plastics, *e*.*g*., polyethylene terephthalate (PET), polyethylene terephthalate, glycol modified (PETG), polyimide, polycarbonate, polystyrene, silicon, ceramic, glass, and the like.

A feature of the subject electrochemical test strips is that the working and reference electrodes as described above generally face each other and are separated by only a short distance, such that the distance between the working and reference electrodes in the reaction zone or area of the electrochemical test strip is extremely small. This minimal spacing of the working and reference electrodes in the subject test strips is a result of the presence of a thin spacer layer positioned or sandwiched between the working and reference electrodes. The thickness of this spacer layer may range from 50 to 750 µm and is often less than or equal to 500 µm, and usually ranges from about 100 to 175 µm. The spacer layer is cut so as to provide a reaction zone or area, where in many embodiments the volume of the reaction area or zone typically ranges from about 0.1 to 10 µL, usually from about 0.2 to 5.0 µL. The spacer layer may have a circular reaction area cut with side inlet and outlet vents or ports, or other configurations, *e*.*g*., square, triangular, rectangular, irregularly shaped reaction areas, etc. The spacer layer may be fabricated from any convenient material, where representative suitable materials include polyethylene terephthalate (PET), polyethylene terephthalate, glycol modified (PETG), polyimide, polycarbonate, and the like, where the surfaces of the spacer layer may be treated so as to be adhesive with respect to their respective electrodes and thereby maintain the structure of the electrochemical test strip.

In many embodiments, a reagent system or composition is present in the reaction area, where the reagent system interacts with components in the fluid sample during the assay. Reagent systems of interest typically include a redox couple.

The redox couple of the reagent composition, when present, is made up of one or more redox couple agents. A variety of different redox couple agents are known in the art and include: ferricyanide, phenazine ethosulphate, phenazine methosulfate, pheylenediamine, 1-methoxy-phenazine methosulfate, 2,6-dimethyl-1,4-benzoquinone, 2,5-dichloro-1,4-benzoquinone, ferrocene derivatives, osmium bipyridyl complexes, ruthenium complexes, and the like. In many embodiments, redox couples of particular interest are ferricyanide, and the like.

Other reagents that may be present in the reaction area include buffering agents, *e*.*g*., citraconate, citrate, malic, maleic, phosphate, "Good" buffers and the like. Yet other agents that may be present include: divalent cations such as calcium chloride, and magnesium chloride; surfactants such as Triton, Macol, Tetronic, Silwet, Zonyl, and Pluronic; stabilizing agents such as albumin, sucrose, trehalose, mannitol, and lactose.

Examples of such a reagent test strips suitable for use with the subject invention include those described in EP-A-1 067 384, WO 01/57510 and WO 01/57238.

Generally for electrochemical assays, an electrochemical measurement is made using the reference and working electrodes. The electrochemical measurement that is made may vary depending on the particular nature of the assay and the device with which the electrochemical test strip is employed, *e*.*g*., depending on whether the assay is coulometric, amperometric or potentiometric. Generally, the electrochemical measurement will measure charge (coulometric), current (amperometric) or potential (potentiometric), usually over a given period of time following sample introduction into the reaction area. Methods for making the above described electrochemical measurement are further described in U.S. Patent Nos.: 4,224,125; 4,545,382; and 5,266,179; as well as WO 97/18465; WO 99/49307; the disclosures of the priority documents of which are herein incorporated by reference. Regardless of the type of measurement, an electrochemical measurement or signal is made in the reaction zone of the test strip.

Following detection of the electrochemical measurement or signal generated in the reaction zone as described above, the amount of the analyte present in the sample introduced into the reaction zone is then determined by relating the electrochemical signal to the amount of analyte in the sample. Representative meters for automatically practicing these steps are further described in EP-A-1 067 384, WO 01/57510 and WO 01/57238.

Of course, in those embodiments using a colorimetric assay system, a spectrophotometer or optical meter will be employed, where representative meters are further described in, for example, U.S. Patent Nos. 4,734,360; 4,900,666; 4,935,346; 5,059,394; 5,304,468; 5,306,623; 5,418,142; 5,426,032; 5,515,170; 5,526,120; 5,563,042; 5,620,863; 5,753,429; 5,573,452; 5,780,304; 5,789,255; 5,843,691; 5,846,486; 5,968,836 and 5,972,294; the disclosures of which are herein incorporated by reference.

As described above, a feature of the subject invention is the presence of a plurality of skin-piercing elements, *e.g.,* micro-needles or the like, for piercing and penetrating the skin of a patient or user of the device, where such a plurality of skin-piercing elements is directly attached or affixed to the test strip, *i.e.,* the plurality of skin-piercing elements are integrated directly into the test strip. In other words, the plurality of skin-piercing elements are attached directly to the test strip, where in some embodiments the plurality of skin-piercing elements is formed of the same material as at least a portion of the test strip, *i.e.,* as a single piece of material or a unitary construction. As will be described below, the plurality of skin-piercing elements are associated, *i.e.,* are in fluid communication with, at least one fluid pathway, where such a fluid pathway may be a fluid pathway internal to the skin-piercing element (an internal lumen) or may be a fluid pathway in the test strip.

The number of skin-piercing elements affixed to a test strip may vary depending on a variety of factors, including the particular analyte of interest and the particular site on the patient from which the sample is to be obtained. In many embodiments, the number of skin-piercing elements ranges from about 2 to 1000, usually from about 3 to 100 and more usually from about 3 to 10. As will be apparent to those of skill in the relevant art, employing a plurality of micro-piercing elements instead of a singular, larger element, *e*.*g*., a single needle, advantageously minimizes the pain associated with obtaining a sample.

Regardless of the type or position of the plurality of micro-needles, the micro-needles will typically be manufactured of a biocompatible material, typically material which can impart the desired rigidity for piercing and penetrating skin and obtaining sample without breaking or substantially flexing. Materials suitable for use in the subject invention include, but are not limited to, stainless steel, plastics such as polyetherimide, polycarbonate, polyetheretherketone, polyimide, polymethylpentene, polyvinylidene fluoride, polyphenylsulfone and liquid crystalline polymer. In many embodiments, the above mentioned materials may further include particles, *e*.*g*., micro or nano particles or fibers, of a suitable metal, carbon siliceous material, *e*.*g*., glass, or ceramic. In certain embodiments, *e*.*g*., where the plurality of skin-piercing elements is positioned substantially parallel to the test strip, the plurality of skin-piercing elements may be made of the same material as the test strip. In other words, the plurality of skin-piercing elements may be formed of the electrode or insulating material of the test strip such that the plurality of skin-piercing elements and the test strip component or material from which it is formed are one piece or one unit.

The dimensions of the micro-needles may vary depending on a variety of factors such as the type of physiological sample to be obtained, the desired penetration depth and the thickness of the skin layers of the particular patient or user being tested. Generally, the skin-piercing elements are constructed to provide skin-piercing and fluid extraction functions and thus will be designed to be sufficiently robust to withstand insertion into and withdrawal from the skin. Typically, to accomplish these goals, the ratio of the length to diameter (where such diameter is measured at the base of the skin-piercing element) of each skin-piercing element is about 1 to 50, usually about 1 to 10 and more usually about 1 to 5. The length, as measured from the base of the test strip to its distal tip is typically about 1 to 5000 microns, usually about 1 to 3000 microns and more usually about 1 to 2000 microns. Although in certain embodiments the diameters of the micro-needles vary along their lengths, generally the outer diameter of each micro-needle is less than about 400 microns and more usually less than about 200 microns. In many embodiments, the outer diameter of the tip is generally less than about 20 microns and more typically less than about 1 micron. The distance between the skin-piercing elements generally ranges from about 200 to 6000 microns, usually about 200 to 3000 microns and more usually about 200 to 400 microns.

An array of skin-piercing elements may include a mixture of skin-piercing elements having, for example, various lengths, outer diameters, inner diameters, cross-sectional shapes, spacings between the skin-piercing elements and may be hollow or solid, as will be described below.

The plurality of skin-piercing elements may be positioned in any convenient configuration, relative to the test strip, where factors for determining such position may include the configuration of surface area of the patient's skin into which the plurality of skin-piercing elements will be penetrated, the analyte of interest, and the like.

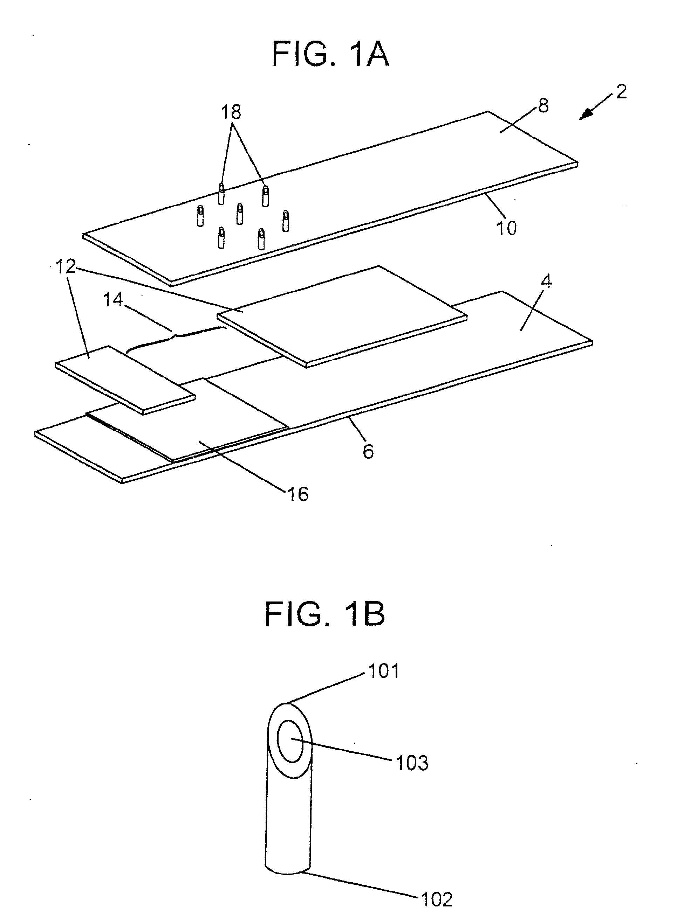

In one embodiment of interest in the subject invention, the plurality of skin-piercing elements, *e.g.,* micro-needles, is positioned to be substantially transverse to, and affixed to the test strip (see Figs 1A-2C). Accordingly, the micro-needles or the like are positioned at about a 90° angle to the test strip and attached thereto. For example, the proximal ends of the micro-needles are made to extend through one of the electrodes at about a 90° angle and more specifically through an electrode and the associated inert support material, such that the plurality of micro-needles protrude out from one side or the other of the test strip, depending on which electrode is used to retain the plurality of micro-needles.

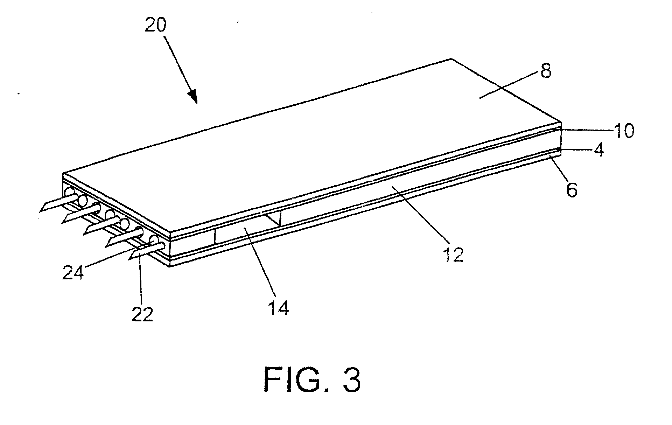

In another embodiment of interest in the subject invention, the plurality of skin-piercing elements, *e*.*g*., micro-needles, is positioned to be substantially parallel to the test strip (see Fig. 3). In other words, the plurality of skin-piercing elements is positioned such that they lie in the same horizontal plane as the test strip, *e*.*g*., the plurality of skin-piercing elements extend from an end of the test strip.

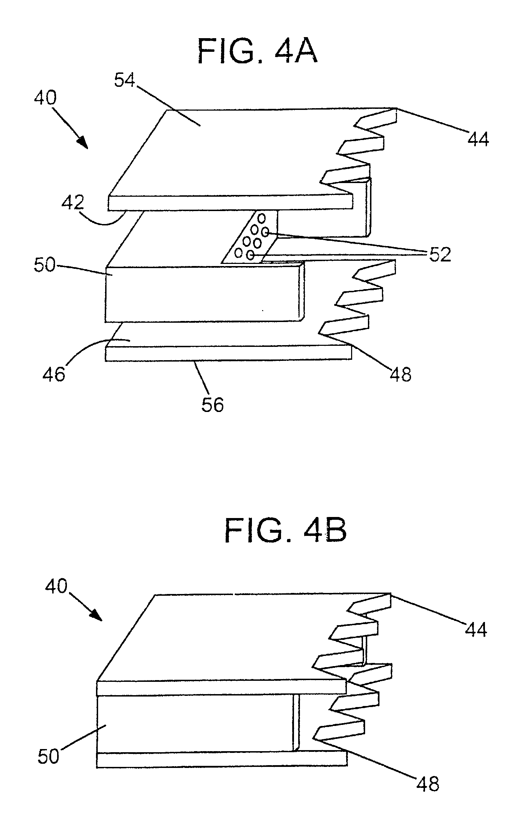

In certain embodiments, the plurality of skin-piercing elements makes up sets of parallel skin-piercing elements, *e*.*g*., a first set of a plurality of skin-piercing elements oppose and are parallel to a second set of a plurality of skin-piercing elements, as will be further described below (see Figs. 4A-4B).

As described above, the test strips of the subject invention include a reaction area or zone defined by the working electrode, the reference electrode and the spacer layer, where the working and reference electrodes define the top and bottom of the reaction zone and the spacer layer defines the walls (or is defined by the matrix of a colorimetric test strip). A feature of the subject invention is that at least one fluid pathway is configured or positioned to be in communication with the reaction area or zone of the test strip to transfer sample thereto, where such a pathway may include an internal lumen of a skin-piercing element or may be a pathway adjacent a skin-piercing element having openings on the surface of the test strip and which runs through the test strip. In other words, the fluid pathways of the present invention are configured to transfer or transport physiological fluid to the reaction area of the test strip, regardless of what type of pathway is included.

As described above, in certain embodiments, the at least one fluid pathway is an internal lumen of the skin-piercing elements, *i*.*e*., the skin-piercing elements are hollowed or bored elements, such that physiological sample may be obtained *in situ* and through which the physiological sample may travel to the reaction area of the test strip. As such, each of the plurality of hollow micro-needles includes a proximal and distal end and a substantially annular bore or lumen therethrough, *i*.*e*., a fluid pathway therethrough. The distal end of each micro-needle extends perpendicular or parallel relative to the test strip and the proximal end is in communication with the reaction area, *i*.*e*., the proximal end terminates at or near the reaction area.

The diameter of the internal fluid pathway or internal lumen of the skin-piercing element may vary depending on a variety of factors. In many embodiments, the diameter of the lumen is substantially constant throughout the entire length, yet in other embodiments the diameter may increase or decrease throughout the length of the lumen to enable sample flow modulation, *e*.*g*., to increase or decrease the rate and/or volume of sample flow through the lumen, as described below. Generally, the inner diameters of the internal lumens are so dimensioned as to exert a capillary force on the physiological fluid. Specifically, the inner diameters of the internal lumens are about 10% to 90% of the outer diameter of a micro-needle (the outer diameter as measured at its base) and thus are usually about 2 to 400 microns, usually about 100 to 250 microns and more usually about 150 to 200 microns.

Regardless of the diameter of the lumen of the micro-needles, the plurality of hollow micro-needles is positioned substantially near or above the reaction area of the test strip, or at least the proximal ends of each micro-needle terminate at or near the reaction area so that sample can flow through the internal pathway to the reaction area. For example, in the embodiment having transversely positioned micro-needle elements, the plurality of micro-needles traverses the entire thickness of an electrode and associated inert support material or inert backing material, into or substantially close to the reaction area. In certain embodiments, the plurality traverses the working electrode, while in others the plurality traverses the reference electrode.

As mentioned above, in other embodiments, at least some of the micro-needles are not hollow and in certain other embodiments none are hollow and are, instead, solid structures associated with pathways or channels, where the pathways or channels are in communication with the reaction area. Specifically, each solid micro-needle is operatively associated with at least one fluid pathway or channel, where the at least one fluid channel has an opening at the surface of the test strip and is in communication with the reaction area of the test strip, *e*.*g*., the at least one fluid pathway ends or terminates at or near the reaction area and, in many embodiments, is positioned substantially within the test strip, as described above. Accordingly, the plurality of solid micro-needles may be positioned directly within a channel, may be laterally adjacent to at least one channel or a test strip may include a combination of both configurations. In those embodiments where the plurality of solid micro-needles are positioned directly within a channel, the plurality may be attached to the test strip by attaching to the inert support material, an electrode, *e*.*g*., an opposing electrode and/or inert support material, or a wall of a channel. In certain embodiments, the test strip may include a combination of hollow-bored and solid micro-needles, where the solid micro-needles may include various fluid pathway configurations, as described above. The number of channels or fluid pathways may vary depending on the particular use of the test strip. As such, there may be more, less or the same number of fluid pathways as there are skin-piercing elements.

In certain embodiments, the solid micro-needles are formed at least in part by one or more electrodes of the test strip, typically both electrodes, such that the micro-needles and electrode(s) are a single piece of material. As such, one electrode may form one set of a plurality of micro-needles and another parallel electrode may form another set of a plurality of micro-needles. In this particular embodiment, the micro-needles further include an insulative material, where the insulative material may be the same as or different from the inert backing of the electrode. In this particular embodiment, the fluid channel(s) are positioned between the sets of electrodes in the spacer layer.

Similar to the lumen diameters described above, the diameters of the fluid pathways or channels associated with solid micro-needles may be constant throughout or may increase or decrease throughout to enable sample flow modulation. Generally, the inner diameters of the pathways are so dimensioned as to exert a capillary force on the physiological fluid. Specifically, the inner diameters of the channels are about 2 to 400 microns, usually about 100 to 250 microns and more usually about 150 to 200 microns.

As will be apparent to one of skill in the art, a test strip of the subject invention may include a variety of different types of micro-needles, where some of the micro-needles may differ in dimensions and/or configurations from the others. For example, a test strip may include a combination of hollow micro-needles and solid micro-needles, where the plurality includes micro-needles of varying dimensions and/or cross-sectional shapes and where some solid micro-needles are laterally adjacent to at least one channel or fluid pathway and other solid micro-needles may be positioned directly inside channels or fluid pathways. Of course, any combination of the above is also contemplated by this invention.

Referring now to the drawings, wherein like numbers refer to like components, Figure 1A shows an exploded view of an exemplary embodiment of a test strip according to the subject invention. In this figure, test strip 2 of the subject invention is shown having reference electrode 4 associated with inert support material 6 and working electrode 10 associated with inert support material 8. Sandwiched in between the two electrodes is spacer layer 12, which is configured and dimensioned to allow for reaction area 14, formed by reference electrode 4 associated with one or more suitable reagent 16, working electrode 10 and spacer layer 12. In this embodiment, a plurality of hollow micro-needles 18 are substantially transverse to, and affixed to the test strip, where the plurality is positioned through working electrode 10 and inert support material 8, and which are positioned directly above and terminate at reaction area 14, *i*.*e*., the proximal end of the plurality extends through the working electrode 10 and inert support material 8 and terminates at or near reaction area 14. Figure 1B provides an enlarged view of a single micro-needle 18 which shows the tip 101, base 102 and inner lumen 103.

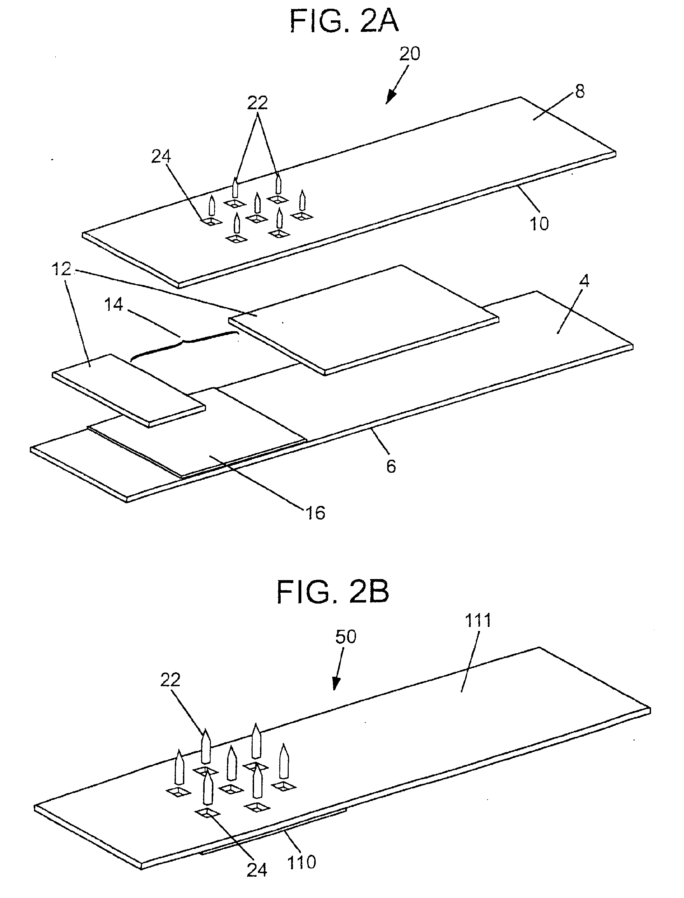

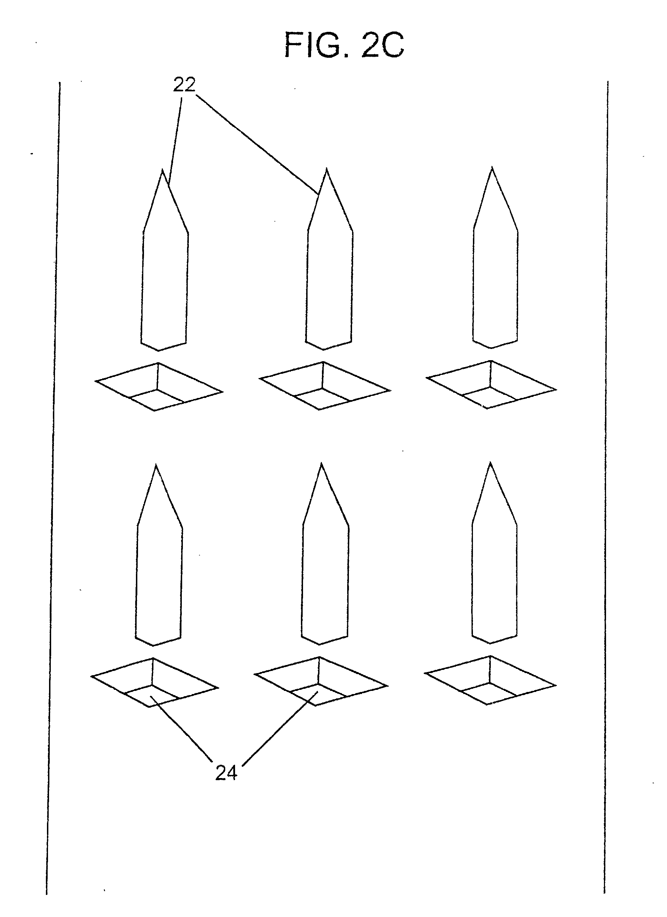

Figure 2A shows an exploded view of another exemplary embodiment of the subject invention illustrated as separate components, where like numerals represent like features. In this embodiment, test strip 20 is shown having a plurality of solid micro-needles 22 attached to inert support material 8, where such a plurality is substantially transverse to, and affixed to the test strip. In turn, each micro-needle is associated with at least one channel or fluid pathway 24, where such fluid pathways are positioned substantially within the test strip 20 and are in communication with reaction area 14, *e*.*g*., they extend or terminate at or around reaction area 14. In this particular embodiment, the plurality of micro-needles 22 are shown laterally adjacent to at least one fluid pathway, however, as described above, some or all of the micro-needles may alternatively be positioned in at least one fluid pathway. An exemplary embodiment of a colorimetric test strip 50 according to the subject invention is provided in Figure 2B, where the colorimetric test strip 50 includes a plurality of solid micro-needles 22 adjacent to fluid pathways 24 and porous matrix 110 attached to solid support 111. Figure 2C shows an enlarged view of the solid-micro-needles 22 and adjacent fluid pathways 24 of Figures 2A and 2B.

Figure 3 shows another exemplary embodiment of the subject invention, where the plurality of skin-piercing elements 22 extends from and is substantially parallel to the test strip 20. For example, the plurality of skin-piercing elements 22 may extend from an end of test strip 20. In this representation, skin-piercing elements are shown as solid structures positioned adjacent to or within fluid pathways 24. As noted above, the parallel micro-piercing elements 22 may alternatively be structures with lumens therethrough such as micro-needles 18 of Figure 1.

Figure 4A shows an exploded view of another exemplary embodiment of the subject invention, wherein the plurality of micro-needles are configured as two spaced apart, parallel sets of skin-piercing elements, wherein the micro-needles of each set are formed by a respective electrode. In other words, a first electrode forms a first set of micro-needles and a second electrode forms a second set of electrodes. Accordingly, Figure 4A shows the individual components of an exemplary embodiment of the subject test strip 40, where the test strip includes a first electrode 42 with a suitable insulating layer 54 and a second electrode 46 with a suitable insulating layer 56, where the two electrodes are separated by a spacer layer 50 having fluid pathways 52 therein. In this particular embodiment, a first set of micro-needles 44 are formed by the first electrode 42 and a second set of micro-needles 48 is formed by the second electrode 46 such that micro-needle sets 44 and 48 are positioned substantially parallel to each other (and the test strip itself) and the physiological sample is thus wicked into the fluid pathways 52 positioned between the sets 44 and 48. It will be obvious to one of skill in the art that the micro-needles will include suitable insulative materials, if and where appropriate, as described above. The test strip 40 also includes a reaction area, as described above (not shown). Figure 4B shows the components of Figure 4A configured as a complete test strip.

### SYSTEMS

As mentioned above, the subject invention includes an analyte concentration determination system capable of obtaining a physiological sample and determining the analyte concentration of an analyte of interest therein, where determining the analyte concentration may be accomplished automatically by an automated device, *e*.*g*., a meter. Accordingly, the analyte concentration determination system of the subject invention includes a test strip having a plurality of skin-piercing elements affixed thereto, as described above, and a meter, where the test strip is configured and adapted to be received by or in the meter.

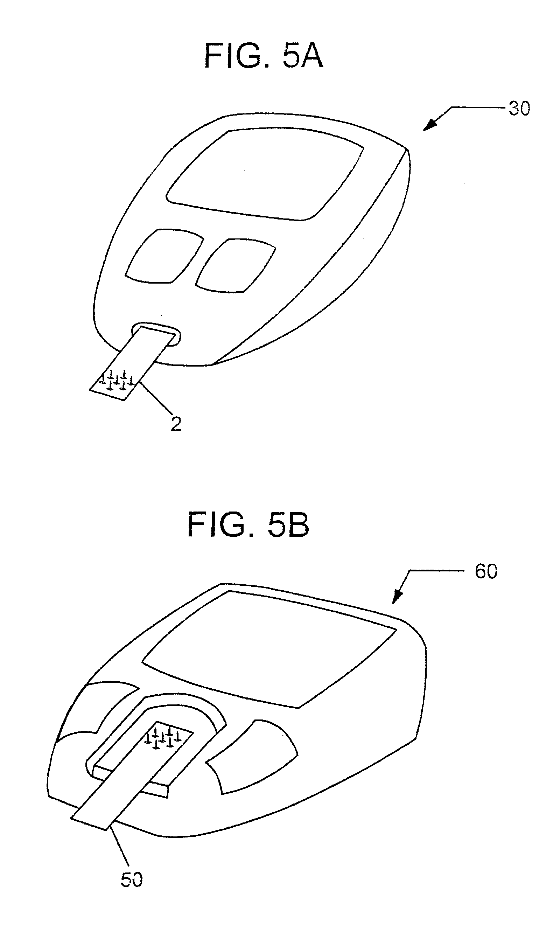

An example of such an analyte concentration determination system is shown in Figures 5A and 5B. Figure 5A illustrates an electrochemical meter 30 having a test strip, *e*.*g*., test strip 2 of Figure 1A, inserted therein and Figure 5B illustrates an optical meter 60 having a colorimetric test strip, *e*.*g*., colorimetric test strip 50 of Figure 2B.

### METHODS

As summarized above, the subject invention also provides methods for practicing the subject invention, where the subject methods advantageously allow for obtaining and determining an analyte concentration of a physiological sample. As noted above, the subject methods find use in the determination of a variety of analyte concentrations, where representative analytes include glucose, cholesterol, lactate, alcohol, and the like. In many embodiments, the subject methods are used to determine the glucose concentration in a physiological sample, *e*.*g*., interstitial fluid, blood, blood fractions, constituents thereof, and the like.

In practicing the subject methods, the first step is to provide a test strip having a plurality of skin-piercing elements, *e*.*g*., micro-needles or micro-skin-piercing elements or the like, affixed thereto, such as the electrochemical test strip described above.

The plurality of micro-needles affixed to the test strip is then inserted into the skin of a patient or user of the subject invention. Depending on the type of physiological sample to be obtained, the skin-piercing elements may penetrate to various skin layers, including the dermis, epidermis and the stratum corneum, but in many embodiments will penetrate into the dermis layer of the skin. Typically, the skin-piercing elements are inserted into the skin, generally into a finger or arm, for about 1 to 60 seconds, usually about 5 to 30 seconds and more usually from about 10 to 15 seconds.

Sample is then collected and transferred to the test strip and specifically to the reaction area of the test strip. In certain embodiments, the sample is collected *in situ*. In those embodiments where the sample is collected *in situ*, the plurality of micro-needles is hollow, or at least one or more micro-needles include a fluid pathway operatively associated with the skin-piercing element, *i*.*e*., an internal lumen therethrough as described above, which is inserted into the skin of the patient such that physiological sample is drawn into the lumens of the plurality of hollow micro-needles. Specifically, the skin is pierced by the plurality of micro-needles, where the plurality penetrates to an appropriate layer of skin and draws physiological sample thereinto. In many embodiments, the sample is drawn into the lumen by a capillary force exerted on the sample and transferred to a reaction area of the test strip.

In other embodiments where some or all of the plurality of micro-needles are solid, sample is collected and transferred by channels or fluid pathways operatively associated with the solid micro-needles which are adjacent or are next to the solid micro-needles. As such, the plurality of skin-piercing elements pierces the skin and penetrates to the appropriate layer of skin. Physiological sample, *e*.*g*., blood, blood fractions, interstitial fluid or the like, formed or expressed at the opening or incision of the skin is collected and transferred to a reaction area of the test strip by fluid pathways or channels with openings on the surface of the test strip, where such channels are in communication with the reaction area and which contact the physiological sample at the surface of the skin. In many embodiments, the sample is drawn into the channels or fluid pathways by a capillary force exerted on the physiological sample, as described above.

Once sample is delivered to the reaction area of the test strip, the concentration of the analyte of interest is determined. As described above for an electrochemical analyte concentration determination assay, an electrochemical measurement is made using the reference and working electrodes. The electrochemical measurement that is made may vary depending on the particular nature of the assay and the device with which the electrochemical test strip is employed, *e*.*g*., depending on whether the assay is coulometric, amperometric or potentiometric. Generally, the electrochemical measurement will measure charge (coulometric), current (amperometric) or potential (potentiometric), usually over a given period of time following sample introduction into the reaction area. Methods for making the above described electrochemical measurement are further described in U.S. Patent Nos.: 4,224,125; 4,545,382; and 5,266,179; as well as WO 97/18465; WO 99/49307; the disclosures of the priority documents of which are herein incorporated by reference. Regardless of the type of measurement, an electrochemical measurement or signal is made in the reaction zone of the test strip.

Following detection of the electrochemical measurement or signal generated in the reaction zone as described above, the amount of the analyte present in the sample introduced into the reaction zone is then determined by relating the electrochemical signal to the amount of analyte in the sample.

In many embodiments, the above described determination and relation processes are performed by an automated device, *e*.*g*., a meter, as is well known in the relevant art. In other words, the test strip is inserted into a meter and the concentration of at least one analyte is determined automatically. Representative meters for automatically practicing these steps are further described in EP-A-1 067 384, WO 01/57510 and WO 01/57238.

### KITS

Also provided by the subject invention are kits for use in practicing the subject methods. The kits of the subject invention include at least one subject test strip, oftentimes a plurality of test strips. The kits may also include a reusable or disposable meter that may be used with reusable or disposable test strips of the kit or from other kits or the subject invention. Certain kits may include various types of test strips, *e*.*g*., where various test strips contain the same or different reagents, *e*.*g*., electrochemical and/or colorimetric test strips, the same or different skin-piercing element configurations, e.g., substantially parallel and/or perpendicular, and the same or different skin-piercing element types, e.g., hollow and/or solid. Finally, the kits may further include instructions for using the subject test strips in the determination of an analyte concentration in a physiological sample. These instructions may be present on one or more of the packaging, a label insert, containers in the kits, and the like.

It is evident from the above description and discussion that the above described invention provides a simple, quick and convenient way to obtain a physiological sample and determine an analyte concentration thereof. The above described invention provides a number of advantages, including ease of use, efficiency, minimal pain and compatibility with both electrochemical and colorimetric analyte concentration determination assays. As such, the subject invention represents a significant contribution to the art.

All publications and patents cited in this specification are herein incorporated by reference as if each individual publication or patent were specifically and individually indicated to be incorporated by reference. The citation of any publication is for its disclosure prior to the filing date and should not be construed as an admission that the present invention is not entitled to antedate such publication by virtue of prior invention.

Although the foregoing invention has been described in some detail by way of illustration and example for purposes of clarity of understanding, it is readily apparent to those of ordinary skill in the art in light of the teachings of this invention that certain changes and modifications may be made thereto without departing from the spirit or scope of the appended claims.

## Claims

1. A test strip for determining at least one target analyte concentration of a physiological sample, said test strip comprising a plurality of skin-piercing needles affixed thereto,
wherein said test strip is configured to be received into a meter.

2. The test strip according to claim 1, wherein said plurality of skin piercing elements is substantially transverse to said test strip.

3. The test strip according to claim 1, wherein said plurality of skin piercing elements is substantially parallel to said test strip.

4. The test strip according to claim 1, wherein said test strip is further comprised of two electrodes separated by a spacer layer, wherein at least one electrode is associated with an inert support material.

5. The test strip according to claim 4, wherein said plurality of skin-piercing elements is attached to said inert support material.

6. The test strip according to claim 4, wherein said plurality of skin-piercing elements is formed by said inert support material.

7. The test strip according to claim 4, wherein said plurality of skin-piercing elements is formed by at least one electrode.

8. The test strip according to claim 4, wherein said plurality of skin-piercing elements is formed by said two electrodes.

9. The test strip according to claim 8, wherein said skin-piercing elements comprise two spaced apart, parallel sets of skin-piercing elements.

10. The test strip according to claim 4, wherein said plurality of skin-piercing elements is formed by said spacer layer.

11. The test strip according to claim 3, further comprising at least one fluid pathway operatively associated with said plurality of skin-piercing elements.

12. The test strip according to claim 11, wherein said at least one of said plurality of skin-piercing elements is laterally adjacent to said at least one fluid pathway.
